# EUROPEAN PATENT APPLICATION

(11) **EP 2 000 134 A1**
(43) Date of publication of application: **10.12.2008**
(21) Application number: 07109761.2
(22) Date of filing: 06.06.2007
(51) Int. Cl.: A61K 31/00, A61K 31/16, A61K 31/198, A61K 31/275, A61K 31/366, A61K 31/401, A61K 31/4196, A61K 31/44, A61K 31/4402, A61K 31/4406, A61K 31/444, A61K 39/00, A61P 25/00, A61P 25/02

(54) **Use of a substance for the improvement of pns lesions**

(71) Applicant: Neuraxo Biopharmaceuticals GmbH, 40699 Erkrath (DE)
(72) Inventor: Masanneck, Carmen, DE-53227 Bonn (DE); Hermanns, Susanne, DE-40274 Hilden (DE)
(74) Representative: von Kreisler Selting Werner

(57) **Abstract**

The present invention relates to the use of a substance for manufacturing a medicament for the improvement of axonal regeneration of PNS tissue or healing of PNS tissue after lesions, wherein the substance is capable of inhibiting or preventing fibrous scar formation.

## Description

The present invention relates to the use of a substance for manufacturing a medicament for the improvement of axonal regeneration of PNS tissue or healing of PNS tissue after lesions, wherein the substance is capable of inhibiting or preventing fibrous, in particular collagenous, scar formation.

The peripheral nervous system (PNS), in contrast to the CNS, is marked by relatively successful regeneration, especially when the nerve is not completely severed (neurotmesis). In contrast to axotomized CNS neurons, injured PNS neurons are able to initiate and maintain a gene expression program that promotes axon outgrowth during the time they regenerate. Attempts are made to optimize the outcome by surgically repairing the damaged nerve, but functional recovery is variable and incomplete.

Approximately 100,000 patients undergo neurosurgery of the peripheral nervous system in the United States of America and Europe each year. Direct suturing, either epineural or perineural, and interpositioning of autologous nerve grafts are the current standard techniques to repair peripheral nerve defects. Typical symptoms are sensory and motor defects that could result in complete paralysis of an affected extremity or development of severe neuropathic pain. Pain symptoms can also result from aberrant axonal regrowth and neuroma formation after a nerve has been injured (Schlosshauer et al., 2006).

Nerve lesions are caused primarily by accidents, tumors, infections, or the iatrogenic side effects of various surgeries, such as orthopedic interventions or aesthetic facial surgery, which could result in facial paralysis (Crawley and Dellon, 1992), (Kretschmer et al., 2001).

Recovery of an injured peripheral nerve is limited by the development of scar tissue at the repair site, representing a mechanical barrier to axonal regeneration, shrinkage of the endoneurial sheaths that contain the nerve fibres, and initiating the development of a 'neuroma' containing multiple branched axon terminals (Lane et al., 1978).

The surgical treatment of nerve injuries still remains a challenge for the surgeon since postoperative results are poor and functional outcomes are far from being satisfactory. The process of scarification at the suture sites represents the main problem for this situation since regenerating axons have to pass this location in order to reach the end organ.

EP 0981549 discloses the use of an inhibitor substance for the improvement of neuronal regeneration of injuries to adult mammalian CNS caused by lesions. The inhibitor substances were capable of inhibiting the synthesis of basal membrane formation, which is hypothesized as a crucial impediment for regeneration.

GB 2304045 discloses a composition for promoting the healing of wounds with reduced scarring. Particularly, the composition comprises TGF-β. The composition was tested for the effects upon wound healing of skin wounds. The results showed that the wounds treated with the composition appeared to be accelerated in their healing compared to control wounds. The inventors suppose that the composition improves the healing of CNS injuries.

Atkins et al. investigated the effect of scarring at a site or peripheral nerve repair by comparing regeneration of the sciatic nerve in mice. It has been found that higher levels of collagen scar formation were associated with smaller compound action potentials, slower conduction velocities and a reduction in fibre numbers across the repair site. A correlation between the level of scarring at a peripheral nerve repair site and the extend of recovery is disclosed. Furthermore, it is shown that the area of the scar was significantly greater in mice with increased propensity for scarring. It has been hypothesized that scarring impedes regeneration at sites of nerve repair and that therapeutic application of scar-preventing agents might be useful. However, experimental evidence for inhibiting agents was not shown (Atkins et al., 2006).

One object of the invention was to improve axonal regeneration of PNS tissue or healing of PNS tissue after lesion.

Surprisingly, improvement of axonal regeneration of PNS tissue or healing of PNS tissue after lesion is achieved by the use of a substance for manufacturing a medicament for the improvement of PNS lesions, wherein the substance is capable of inhibiting or preventing fibrous, in particular collagenous, scar formation.

According to the invention the substance is selected from a group consisting of antibodies against collagen IV, laminin, entactin; Fe-chelating agents; inhibitors of amino acids hydroxylases, such as prolyl-4-hydroxylase, lysyl-hydroxylase; 2-oxoglutarate competitors; antisense oligo nucleotides or oligo nucleotide analogs; small interfering RNAs (siRNAs); aptamers.

In one embodiment, the substance is selected from a group consisting of N-oxaloglycine; pyridine derivatives, such as 5-arylcarbonylamino or 5-arylcarbamoyl-derivatives, 2-carboxylate, 2,5-dicarboxylate or ethyl esters or ethyl amides or -5-acylsulfonamides thereof, 2,4-dicarboxylate, ethyl esters or ethylamides or dimethoxyethylamides thereof; 3,4'-bipyridine, such as 5-amino-6-(1H)-one, 1,6-dihydro-2-methyl-6-oxo-5-carbonitril; 2,2'-bipyridine, such as 5,5'-dicarboxylic acid ethyl ester or ethyl amide; 3,4'-dihydroxybenzoate, such as the diethyl ester; proline and its structural and functional analoges; β-aminopropionitrile; de(s)ferrioxamine; de(s)ferasirox; anthracyclines; 2,7,8-trihydroxy anthraquinones, fibrostatin-C; coumalic acid or its salts; 5-oxaproline, β-lactam antibiotics.

In one embodiment, the substance according to the invention is applied locally to the PNS. Particularly, the substance is applied systemically or orally.

In one embodiment of the present invention, a formulation consists of an iron-chelating agent in a sustained release lipid formulation.

In a further embodiment the substance for manufacturing a medicament is de(s)ferrioxamine and/or de(s)ferasirox, in particular de(s)ferrioxamine.

Perineurin is the name of the medicament comprising de(s)ferrioxamine. The term deferoxamine is a synonym.

### Brief Description of the figures

Fig.1: In situ picture of the transected and re-sutured rat median nerve. 10 µl Perineurin have been applied to the suture site. A piece of the external jugular vein is wrapped around the suture site and provides that the inhibitor stays in place.
Fig. 2: Grasping force test. Animals that received the substance recovered grasping force significantly earlier over time and in-between groups. Data were analyzed using an ANOVA (GLM) with the factors Treatment and Time with Time as a repeated-measures factor. Recovery of Perineurin treated animals is significant over time and in between groups.
Fig. 3: Final soleus muscle weight (wet). Perineurin treated animals have significantly higher muscle weights compared to any control group. Data were analyzed using an one factorial ANOVA for the factor Treatment. For post-hoc comparison between the four experimental groups a Student-Newman-Keuls test was used.
Fig. 4: Immunohistochemical staining for RT97 (neurofilament) in coronal sections taken from the part of the median nerve distal to the lesion site. Perineurin treated animals show more regenerated neurofilament positive structures in the distal nerve stump compared to controls.

### Example

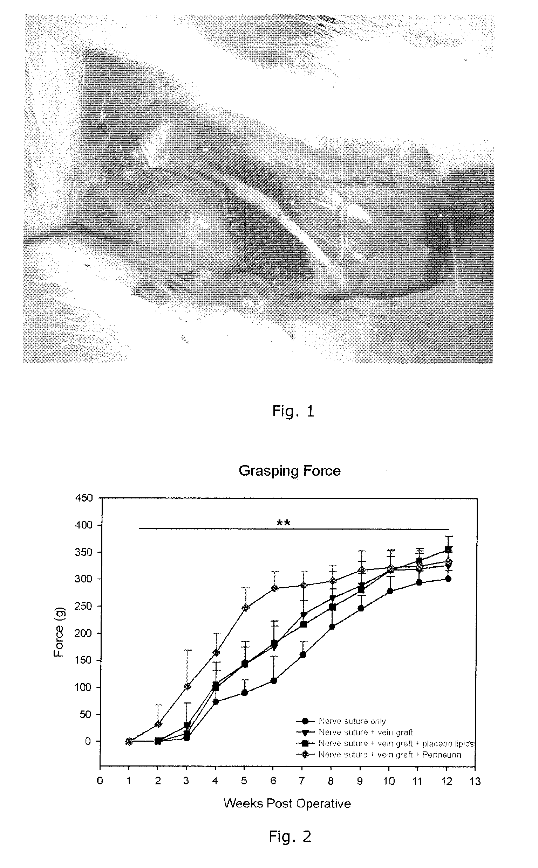

For evaluation of scar formation and inhibition we used 30 female Wistar rats, weighing between 220 and 240 grams at the time of the operation. The median nerve was exposed at the axilla and the nerve was cut at the site where the brachial artery crosses the median nerve. The injury was supplied with a primary nerve suture using an 11-0 microsuture under an operating microscope (group 1, n = 12). In another group we compared the impact of the scar inhibitor using 10 µl of the medicament Deferoxamine mesilate (de(s)ferrioxamine; mesilate characterizes the pharmaceutical form) in a sustained release lipid formulation (c=200 mg/g; lipids consisting of phosphatidylcholine and phosphatidylglycerol in a 1:2 ration) at the suture site of the median nerve (group 4, n = 12). In order to avoid a dilution of the inhibitor in the surrounding tissue we used the external jugular vein of the animals as a depot that was wrapped around the suture sites (Fig. 1).

To close small spaces between veins and nerve both ends of the vein were sutured with 11-0 around the nerve, stitching only superficially the epineurium. To create a control group another twelve animals were supplied with the wrapped around vein but without the introduction of the scar inhibitor (group 2, n = 12). For further control reasons we used the lipid solution (10 µl) that was added for pharmacological reasons to the original scar inhibitor without the active generic drug in a venous depot around the suture site (group 3, n = 12). The animals were observed for 12 weeks. Their functional regeneration was assessed during this time by admitting them to the grasping test. The grasping test was performed once a week measuring the force of the animals in their forepaw as an indicator of the median nerve regeneration.

Animals that received the substance recovered grasping force significantly earlier over time and in-between groups (Fig. 2).

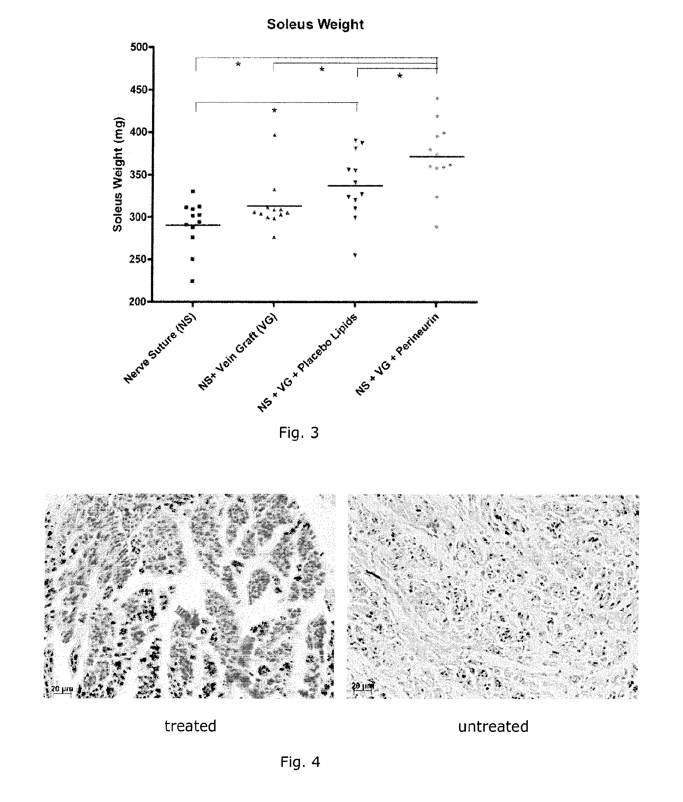

Moreover, the study shows that the substances according to the invention increase the target muscle weight. Animals that received the substance showed significantly lesser atrophy of the target muscle (M. soleus) (Fig. 3).

Furthermore, after the observation period the animals were electrophysiologically monitored measuring the nerve conduction velocity, the latency and the compound muscle action potential of the end organ (flexor digitorum sublimes muscle). Treated animals showed significantly better results in physiological parameters compared to controls (data not shown). Afterwards, the animals were sacrificed and the target muscle (M. soleus) were weighed. Moreover the rats were perfused and the specimens were gathered from the suture sites and the muscle in order to perform histomorphometric analysis of the median nerve (fibre number, fibre diameter, myelin thickness, total number of axons, data not shown). Treated rats showed significantly more axons distal to the lesion site, that are positively stained for the immunohistochemical marker RT97, staining neurofilament, compared to control animals (Fig. 4).

### Reference List

1. Atkins S, Smith KG, Loescher AR, Boissonade FM, O'Kane S, Ferguson MW, Robinson PP (2006) Scarring impedes regeneration at sites of peripheral nerve repair. Neuroreport 17: 1245-1249.
2. Crawley WA, Dellon AL (1992) Inferior alveolar nerve reconstruction with a polyglycolic acid bioabsorbable nerve conduit. Plast Reconstr Surg 90: 300-302.
3. Kretschmer T, Antoniadis G, Braun V, Rath SA, Richter HP (2001) Evaluation of iatrogenic lesions in 722 surgically treated cases of peripheral nerve trauma. J Neurosurg 94: 905-912.
4. Lane JM, Bora FW, Jr., Pleasure D (1978) Neuroma scar formation in rats following peripheral nerve transection. J Bone Joint Surg Am 60: 197-203.
5. Schlosshauer B, Dreesmann L, Schaller HE, Sinis N (2006) Synthetic nerve guide implants in humans: a comprehensive survey. Neurosurgery 59: 740-747.

## Claims

1. Use of a substance for manufacturing a medicament for the improvement of axonal regeneration of PNS tissue or healing of PNS tissue after lesion,
wherein the substance is capable of inhibiting or preventing fibrous scar formation.

2. The use of a substance according to claim 1, wherein the substance is selected from the group consisting of antibodies against collagen IV, laminin, entactin; Fe-chelating agents; inhibitors of amino acids hydroxylases, such as prolyl-4-hydroxylase, lysine-hydroxylase; 2-oxoglutarate competitors; antisense oligo nucleotides or oligo nucleotide analogs; small interfering RNAs (siRNAs); aptamers.

3. The use of a substance according to claim 2, wherein the substance is selected from the group consisting of N-oxaloglycine; pyridine derivatives, such as 5-arylcarbonylamino or 5-arylcarbamoyl-derivatives, 2-carboxylate, 2,5-dicarboxylate or ethyl esters or ethyl amides or -5-acylsulfonamides thereof, 2,4-dicarboxylate, ethyl esters or ethylamides or dimethoxyethylamides thereof; 3,4'-bipyridine, such as 5-amino-6-(1H)-one, 1,6-dihydro-2-methyl-6-oxo-5-carbonitril; 2,2'-bipyridine, such as 5,5'-dicarboxylic acid ethyl ester or ethyl amide; 3,4'-dihydroxybenzoate, such as the diethyl ester; proline and its structural and functional analoges; β-aminopropionitrile; de(s)ferrioxamine; de(s)ferasirox; anthracyclines; 2,7,8-trihydroxy anthraquinones, fibrostatin-C; coumalic acid or its salts; 5-oxaproline, β-lactam antibiotics.

4. The use of a substance according to any one of the preceding claims, wherein the substance is applied locally, systemically or orally to the PNS.
